# EUROPEAN PATENT APPLICATION

(11) **EP 4 386 090 A1**
(43) Date of publication of application: **19.06.2024**
(21) Application number: 22306850.3
(22) Date of filing: 12.12.2022
(51) Int. Cl.: C12Q 1/6886, C12Q 1/689, A61K 31/00, A23L 33/00, A61P 31/04, A61K 35/74, C12N 7/00

(54) **METHOD FOR DETERMINING AND IMPROVING THE POTENTIAL EFFICACY OF ANTICANCER TREATMENT**

(71) Applicant: ASSISTANCE PUBLIQUE - HÔPITAUX DE PARIS, 75012 Paris 12 (FR); INSTITUT NATIONAL DE RECHERCHE POUR L'AGRICULTURE, L'ALIMENTATION ET L'ENVIRONNEMENT, 75007 Paris (FR); UNIVERSITE PARIS CITE, 75006 Paris (FR); INSTITUT NATIONAL DE LA SANTE ET DE LA RECHERCHE MEDICALE (INSERM), 75013 Paris (FR)
(72) Inventor: MICHONNEAU, David, 78230 Le Pecq (FR); VALLET, Nicolas, 37000 Tours (FR); LEPAGE, Patricia, 94110 Arcueil (FR); LE GOFF, Jérome, 75010 PARIS (FR)
(74) Representative: Gevers & Orès

(57) **Abstract**

The present invention relates to the field of anticancer treatment, more specifically to methods for *ex vivo* determining whether a patient with hematological malignancies is likely to benefit from allogeneic hematopoietic stem cell transplantation (allo-HSCT) by analyzing the gut microbiota in a fecal sample from said patient; the present invention also relates to treatments aimed to reduce the risk of relapse following allo-HSCT.

## Description

The present invention relates to the field of anticancer treatment, more specifically to methods for *ex vivo* determining whether a patient with hematological malignancies is likely to benefit from allogeneic hematopoietic stem cell transplantation (allo-HSCT) by analyzing the gut microbiota in a fecal sample from said patient; the present invention also relates to treatments aimed to reduce the risk of relapse following allo-HSCT.

Allogeneic hematopoietic stem cell transplantation (allo-HSCT) is a curative treatment of hematological malignancies (Copelan, E.A. (2006) Hematopoietic Stem-Cell Transplantation. N Engl J Med 354, 1813-1826). Relapse is the main cause of death following allo-HSCT (Horowitz et al. (2018) Bone Marrow Transplant 53, 1379-1389). The curative graft-versus-tumor (GVT) effect relies on alloreactivity of donor immune cells against tumor cells (Blazar et al. (2020) Nat Rev Clin Oncol 17, 475-492). GVT is mainly mediated by donor T cells, as illustrated by the higher risk of relapse associated with T cell-depleted graft (Horowitz et al. (1990) Blood, 8) and the preventive effects of donor lymphocyte infusions (DLI) (Schmid et al. (2007) JCO 25, 4938-4945). However, allo-HSCT benefits may be counterbalanced by graft-versus-host disease (GVHD), one of the major complications of allo-HSCT in which T cells target host healthy tissues (Horowitz *et al.* (2018); Cho et al. (2012) Biology of Blood and Marrow Transplantation 18, 1136-1143; Zeiser, R., and Blazar, B.R. (2017) N Engl J Med 377, 2167-2179).

Lung chronic GVHD (cGVHD) occurs in 10% of patients in the first 2 years after HSCT (Bergeron et al. (2018) Eur Respir J 51, 1702617) and shares triggering mechanisms with bronchiolitis obliterans syndrome (BOS) (Barker et al. (2014) N Engl J Med 370, 1820-1828) that occur after lung transplantation. After lung transplantation, azithromycin, a second-generation macrolide, prevents BOS (Vos et al. (2011) European Respiratory Journal 37, 164-172).

In the context of a randomized, multicenter, placebo-controlled, double-blind, superiority study (ALLOZITHRO trial, NCT01959100) aimed to evaluate azithromycin as lung cGVHD prophylaxis, Inventors have conducted an analysis of the fecal and blood samples collected during the allo-HSCT procedure from patients included in the ALLOZITHRO trial.

Gut microbiota is disturbed after allo-HSCT (Shono, Y., and van den Brink, M.R.M. (2018) Nat Rev Cancer 18, 283-295). This dysbiosis is characterized by lower gut microbiota α-diversity and *Enterococcaceae* domination (Jenq et al. (2012) Journal of Experimental Medicine 209, 903-911; Peled et al. (2020) N Engl J Med 382, 822-834). Antibiotics used during and before the procedure influence bacterial taxa abundances and bacteria domination (Shono et al. (2016) Sci. Transl. Med.; Taur et al. (2012) Clinical Infectious Diseases 55, 905-914; Weber et al. (2017) Biology of Blood and Marrow Transplantation 23, 845-852). Parenteral nutrition also influences gut microbiota composition (Jenq et al. (2015) Biology of Blood and Marrow Transplantation 21, 1373-1383). Low microbial diversity is associated with higher risk of death, notably driven by non-relapse mortality (Peled et al. (2020) NEJM 328, 822-834; Taur et al. (2014) Blood 124, 1174-1182). Considering relapse, few studies report the link between gut microbiota and allo-HSCT relapse. Low abundance of *Blautia* was associated with higher risk of relapse (Jenq *et al.* (2015)) while presence of a cluster of operational taxonomic units (OTU), mainly composed of *Eubacterium limosum,* was associated with lower risk of relapse (Peled et al. (2017) JCO 35, 1650-1659). Mechanisms underlying the role of gut microbiota in antitumor responses after allo-HSCT are still unknown, but might be related to gut microbiome derived metabolites (Postler, T.S. and Ghosh, S. (2017) Cell Metabolism 26, 110-130; Yang et al. (2017) Cell Host & Microbe 22, 757-765.e3). It thus remains necessary to better understand the role of gut microbiota in antitumor responses after allo-HSCT to define new complementary therapies for the treatment of hematological malignancies.

In particular, Inventors showed that gut microbiota composition was associated with relapse or remission: *Bacteroides sp. DJF_B097* (relative of *Bacteroides stercoris*) and *Prevotella sp. DJF_RP53* (relative of *Prevotella copri)* and their closest bacterial strains are associated with complete remission whereas *Bacteroides fragilis* (and related strains) is associated with higher risk of relapse.

By applying a multi-omics approach to quantify abundances and diversity of bacteria taxa, viral species and fecal metabolites levels, the Inventors decipher how gut bacteriome, virome and metabolomic profiles interact in patients who received allo-HSCT. They highlighted a network of bacteria, bacteriophages and metabolic pathways associated with post-transplant relapses.

The present invention thus relates to a method for predicting the response of an individual to an allogeneic hematopoietic stem cell transplantation (allo-HSCT), in particular in the context of a hematological malignancies, comprising the steps of:
(i) determining gut microbial phylotypes (OTU for Operational Taxonomic Unit, ASV for Amplicon Sequence Variant or molecular species; i.e any group of 16S rRNA gene coding sequences sharing at least 97% of similarity between each other and hence grouped together in a taxonomic entity; and represented by a selected representative sequence) in an intestinal sample of said individual;
(ii) determining abundance or relative abundance of the phylotype comprising a nucleotide fragment of sequence having at least 97%, preferably 98%, 99% or 100%, identity with denovo9506 of SEQ. ID. N°1 (affiliated to *Bacteroides fragilis*) and optionally determining a relative abundance of at least one of the OTU comprising a nucleotide fragment of sequence having at least 97%, preferably 98%, 99% or 100%, identity with denovo3073 of SEQ. ID. N°2 (affiliated to *Bacteroides sp. DJF_B097*) and/or with denovo9260 of SEQ. ID. N°3 (affiliated to *Prevotella sp. DJF_RP53 X*);
wherein individual having a gut microbiota enriched in said OTU comprising a nucleotide fragment of sequence having at least 97%, preferably 98%, 99% or 100%, identity with denovo9506 of SEQ. ID. N°1 and optionally depleted in at least one of OTU comprising a nucleotide fragment of sequence having at least 97%, preferably 98%, 99% or 100%, identity with denovo3073 of SEQ. ID. N°2 and/or with denovo9260 of SEQ. ID. N°3 has a higher risk of relapse.

According to a specific embodiment, the present invention relates to a method for predicting the response of an individual to an allogeneic hematopoietic stem cell transplantation (allo-HSCT), in particular in the context of a hematological malignancies, comprising the steps of:
(i) determining gut microbial phylotypes (OTU for Operational Taxonomic Unit, ASV for Amplicon Sequence Variant or molecular species; i.e any group of 16S rRNA gene coding sequences sharing at least 97% of similarity between each other and hence grouped together in a taxonomic entity; and represented by a selected representative sequence) in an intestinal sample of said individual;
(ii) determining abundance or relative abundance of the phylotype comprising a nucleotide fragment of sequence having at least 97%, preferably 98%, 99% or 100%, identity with denovo9506 of SEQ. ID. N°1;
wherein individual having a gut microbiota enriched in said phylotype has a higher risk of relapse.

Preferably, said individual may then receive a treatment to modify gut microbiota composition using for example specific bacteriophages.

The "relative abundance" of a phylotype is defined as a percentage of the number of sequences grouped into this phylotype to the total number of filtered sequences in a given sample. Phylotype should group at least two 16S rRNA gene sequences (singletons, i.e. phylotypes containing only one sequence, are not considered).

The determination of gut microbial composition and of the abundance and relative abundance of a given phylotype can be done with classical and appropriate method known by the person skilled in the art. In a particular embodiment, said determination is performed on total DNA extracted from human fecal or mucosal or tissue sample by techniques that are well known by the skilled person such as Shotgun metagenomic sequencing or 16S rRNA gene sequencing. Determination can also be performed by quantitative PCR technology with specific probes targeting either the specific phylotype sequence or its affiliated bacterial isolates and strains and any sequences having at least 97%, preferably 98%, 99% or 100%, identity with the nucleic sequence of said phylotype.

Each phylotype is represented at a different level in relative abundance, some phylotype relative abundance ranging from 0.01 to 0.1% of total number of sequences, some other from 0,5% to 35% of the total number of sequences. Preferably, in the method of the invention, phylotype relative abundance is assessed by applying a detection threshold of the total number of sequences (per sample).

According to another embodiment, the present invention relates to a method for predicting the response of an individual to an allogeneic hematopoietic stem cell transplantation (allo-HSCT), in particular in the context of a hematological malignancies, comprising the steps of:
(i) determining gut microbial phylotype composition in an intestinal sample of said individual;
(ii) determining the presence or the absence of the phylotype comprising a nucleotide fragment of sequence having at least 97%, preferably 98%, 99% or 100%, identity with denovo9506 of SEQ. ID. N°1 by applying a detection threshold of 1% of the total number of sequences (per sample) to define a phylotype's presence or absence, or applying quantitative PCR detection with specific phylotype or bacterial isolate targeting probes;
wherein individual having a gut microbiota showing the presence of said phylotype has a higher risk of relapse.

In another embodiment, the present invention also relates to a method for predicting the response of an individual to an allogeneic hematopoietic stem cell transplantation (allo-HSCT), in particular in the context of a hematological malignancies, comprising the steps of:
(i) determining gut microbial phylotypes (OTU for Operational Taxonomic Unit, ASV for Amplicon Sequence Variant or molecular species; i.e any group of 16S rRNA gene coding sequences sharing at least 97% of similarity between each other and hence grouped together in a taxonomic entity; and represented by a selected representative sequence) in an intestinal sample of said individual;
(ii) determining abundance or relative abundance of at least one phylotype comprising a nucleotide fragment of sequence having at least 97%, preferably 98%, 99% or 100%, identity with denovo3073 of SEQ. ID. N°2 and/or with denovo9260 of SEQ. ID. N°3;
wherein individual having a gut microbiota enriched in at least one said phylotype or in the 2 phylotypes is a good responder to the allo-HSCT.

According to another embodiment, the present invention relates to a method for predicting the response of an individual to an allogeneic hematopoietic stem cell transplantation (allo-HSCT), in particular in the context of an hematological malignancies, comprising the steps of:
(i) determining gut microbial phylotype composition in an intestinal sample of said individual;
(ii) determining the presence or the absence of at least one phylotype comprising a nucleotide fragment of sequence having at least 97%, preferably 98%, 99% or 100%, identity with denovo3073 of SEQ. ID. N°2 and/or with denovo9260 of SEQ. ID. N°3, by applying a detection threshold of 1% of the total number of sequences (per sample) to define a phylotype's presence or absence, or applying quantitative PCR detection with specific phylotype or bacterial isolate targeting probes;
wherein individual having a gut microbiota showing the presence of at least one of the two said phylotypes or the two said phylotypes is a good responder to the allo-HSCT.

As used herein, "hematological malignancies" is a generic term to designate various blood cancers; such cancers are classified by WHO according to their presumed cell of origin, genetic abnormalities and clinical features (see Khoury, J. D. et al. The 5th edition of the world health organization classification of haematolymphoid tumours: Myeloid and histiocytic/dendritic neoplasms. Leukemia 36, 1703-1719 (2022) and Alaggio, R. et al. The 5th edition of the world health organization classification of haematolymphoid tumours : Lymphoid neoplasms. Leukemia 36, 1720-1748 (2022). Non limitative example of hematological malignancies are acute myeloid leukemias, acute lymphoid leukemias, myelodysplastic neoplasms, myeloproliferative neoplasms, B and T non-Hodgkin lymphomas and lymphoproliferative disorders, Hodgkin lymphomas.

Allogeneic hematopoietic stem cell transplantation (allo-HSCT) is a procedure in which a portion of a healthy donor's peripheral blood stem cell or bone marrow stem cell is obtained and prepared for intravenous infusion.

Accordingly, the methods of the invention allow to determine whether a patient with hematological malignancies is likely to benefit from an allogeneic hematopoietic stem cell transplantation (allo-HSCT) by analysis the gut microbiota in an intestinal sample, such as a mucosal sample, for example obtained after a biopsy, or a feces sample, from said patient; this may be assessed before and/or after the allo-HSCT.

In the context of the present invention, "relapse" means the return of the disease, a hematological malignancy, or the signs and symptoms or biological abnormalities (also designed as minimal residual disease (MRD)) of said disease after a period of improvement.

A patient that is a "good responder to the allo-HSCT" is a patient who is affected with a hematological cancer and who has received or will receive an allogeneic hematopoietic stem cell transplantation (allo-HSCT) and will show a clinically significant improvement after receiving said anticancer treatment; the clinically significant improvement may be assessed by clinical examination (body weight, general status, pain and palpable mass, if any), biomarkers and imaging studies (ultrasonography, CT scan, PET scan, MRI) and by marrow examination (bone marrow aspiration or biopsy). In a specific embodiment, a good response to the allo-HSCT is complete remission defined by standard criteria well known by the person skilled in the art.

Gut microbiota relates to the population of microorganisms living in the intestine of any organism belonging to the animal kingdom; in the present invention, said organism is preferably a human. The gut microbial composition evolves throughout the entire life and is the result of different environmental influences.

Dysbiosis refers to the deleterious loss of balance in gut microbial composition that may arise in some specific situations.

The intestinal sample is collected at any moment before deciding the allogeneic hematopoietic stem cell transplantation (allo-HSCT) or at any moment after.

Phylotype in the present invention regroups bacterial 16S rRNA gene sequences sharing the same or similar nucleotidic bases (at least 97% of sequence identity on the targeted 16S region) and is named "denovo#". Each phylotype is affiliated to a bacterial species, either isolated or not, cultured or uncultured when sharing 98% or more sequence similarity with a sequence described in public databases.

Alternatively, the method for predicting the response of an individual to an allogeneic hematopoietic stem cell transplantation (allo-HSCT), in particular in the context of a hematological malignancies, may be conducted by detecting the increase or the decrease of at least one individual's metabolite.

More specifically,
- the decrease of 2-hydrosebacate and/or of 2-methylmalonylcarnitine in an intestinal sample of one individual is associated with a higher risk of relapse;
- the decrease of hercynine, arabinose, glycerophosphoserine, glycerophosphocholine, 1-steaoryl-GPI, glycérophosphoinositol and/or 1-palmitoyl GPI and/or the increase of N-methylalanine and/or dopamine 3-O-sulfate in a plasma or intestinal sample of one individual are associated with a good response to the allo-HSCT.

The present invention also relates to the treatment of a patient diagnosed as having a risk of relapse for modifying its gut microbiota.

According to an embodiment, such treatment is done with bacteriophages.

Bacteriophages (phages) are a class of viruses that specifically lyse bacteria. They are widely present in soil, air, water and living organisms. They have strong specificity and bind to specific sites on the surface of bacterial cells. Since the first discovery of bacteriophages by Frederik Tword in 1915, a growing body of research has demonstrated that bacteriophages have high antimicrobial activity and specificity against drug-resistant bacteria and prevent damage to microbial communities.

Compared with antibiotic treatment, the use of bacteriophages has extremely low side effects and is more rapid and effective, does not inhibit the body's natural immunity or cause allergic reactions, and is not invasive and toxic to humans, other mammals and plants. Of particular relevance, bacteriophage will not kill non-pathogenic, "normal flora" bacteria, thereby retaining the "colonization resistance" of reservoirs such as the human intestinal tract and can be used to specifically target one or several bacterial species.

Accordingly, the present invention relates to at least one phage targeting *Bacteroides fragilis* (directly or indirectly) for preventing cancer relapse in a patient having received an allogeneic hematopoietic stem cell transplantation (allo-HSCT); preferably said cancer is selected in the group consisting of acute myeloid leukemias, acute lymphoid leukemias, myelodysplastic neoplasms, myeloproliferative neoplasms, B and T non-Hodgkin lymphomas and lymphoproliferative disorders, Hodgkin lymphomas.

As used herein, a phage targeting *Bacteroides fragilis* refers to a bacteriophage that has direct or indirect antibacterial activity against *Bacteroides fragilis,* that is to say the ability to kill and/or inhibit the growth or reproduction of the bacteria *Bacteroides fragilis.* Direct antibacterial activity may be assessed by culturing *Bacteroides fragilis* according to known techniques, contacting the culture with a bacteriophage and monitoring bacterial growth and lysis after said contacting. Decreased size of colonies, or decreased total numbers of colonies, indicate a bacteriophage with antibacterial activity against *Bacteroides fragilis.* Indirect effect may be assessed by culturing *Bacteroides fragilis* according to known techniques, contacting said *Bacteroides fragilis* culture with the co-culture of a specific bacteriophage and known bacterial target and monitoring *Bacteroides fragilis* bacterial growth and lysis after said contacting. Decreased size of colonies, or decreased total numbers of colonies, indicate a bacteriophage-bacterial target leading to antibacterial activity against *Bacteroides fragilis.*

Efforts are made to select phages that (i) are lytic, (ii) are specific to *Bacteroides fragilis* and (iii) lyse more than 70% of the *Bacteroides fragilis* and with good resilience throughout human gut tract (i.e. resistance to pH variation, bile and pancreatic salt, as generally evaluated by a simulator of the human intestinal microbial ecosystem (SHIME) Van de Wiele *et al.,* 2015).

Examples of bacteriophage that has direct antibacterial activity against *Bacteroides fragilis* are:
- lytic phages VA7, MTK and UZ-1 of Siphoviridae family;
- bacteriophage B56-3;
- bacteriophage B40-8;
- bacteriophage vB_BfrS_23.

Examples of bacteriophage that has indirect antibacterial activity against *Bacteroides fragilis* are:
- *Lactococcus* phage D4410;
- *Lactococcus* phage D4412;
- *Lactobacillus* phage Lrm1;
- *Streptococcus* phage Dp-1;
- *Rhodococcus* virus Poco6;
- *Alphapapillomavirus* 10;
- *Lactococcus* phage BK5-T.

The isolated bacteriophages may be administered alone or incorporated into a pharmaceutical composition.

The pharmaceutical compositions of the invention may therefore comprise one, two or more isolated bacteriophages with direct or indirect antibacterial activity against *Bacteroides fragilis.*

The present invention thus also relates to a pharmaceutical composition comprising at least one phage targeting *Bacteroides fragilis* for preventing cancer relapse in a patient having received an allogeneic hematopoietic stem cell transplantation (allo-HSCT).

The pharmaceutical compositions comprising at least one bacteriophage can be formulated in a unit dose or multi-dose formulation.

Phages targeting *Bacteroides fragilis* are preferably formulated in pharmaceutical compositions also containing a pharmaceutically acceptable carrier, and can be stored as a concentrated aqueous solution or lyophilized powder preparation. The pharmaceutical composition may contain other components so long as the other components do not reduce the effectiveness of the bacteriophage so much that the therapy is negated. Pharmaceutically acceptable carriers are well known, and one skilled in the pharmaceutical art can easily select carriers suitable for particular routes of administration (Remington's Pharmaceutical Sciences, Mack Publishing Co., Easton, PA, 1985).

Suitable formulations of the pharmaceutical composition can be selected from the group consisting of ointments, solutions, suspensions or emulsions, extracts, powders, granules, sprays, lozenges, tablets or capsules and additionally include a dispersing agent or a stabilizing agent.

In an embodiment, said pharmaceutical composition is formulated for delivery to the intestine (e.g., the small intestine and/or the colon).

Accordingly, the bacteriophage may also be formulated for rectal delivery to the intestine (e.g., the colon). Thus, in some embodiments, the compositions comprising bacteriophage may be formulated for delivery by suppository, colonoscopy, endoscopy, sigmoidoscopy or enema. A pharmaceutical preparation or formulation and particularly a pharmaceutical preparation for oral administration, may include an additional component that enables efficient delivery of the compositions of the disclosure to the intestine (e.g., the colon). A variety of pharmaceutical preparations that allow for the delivery of the compositions to the intestine (e.g., the colon) can be used. Examples thereof include pH sensitive compositions, more specifically, buffered sachet formulations or enteric polymers that release their contents when the pH becomes alkaline after the enteric polymers pass through the stomach. When a pH sensitive composition is used for formulating the pharmaceutical preparation, the pH sensitive composition is preferably a polymer whose pH threshold of the decomposition of the composition is between about 6.8 and about 7.5. Such a numeric value range is a range in which the pH shifts toward the alkaline side at a distal portion of the stomach, and hence is a suitable range for use in the delivery to the colon. It should further be appreciated that each part of the intestine (e.g., the duodenum, jejunum, ileum, cecum, colon and rectum), has different biochemical and chemical environment. For instance, parts of the intestines have different pHs, allowing for targeted delivery by compositions that have a specific pH sensitivity. Thus, the compositions provided herein may be formulated for delivery to the intestine or specific parts of the intestine (e.g., the duodenum, jejunum, ileum, cecum, colon and rectum) by providing formulations with the appropriate pH sensitivity. (See e.g., Villena et al., Int J P harm 2015, 487 (1-2): 314-9).

The phages targeting *Bacteroides fragilis* may be administered orally; in such a case, phages may be incorporated in a tablet or capsule which will enable transfer of phages through the stomach with no reduction of phage viability due to gastric acidity, and release of fully active phages in the small intestine. In some embodiments, the compositions are formulated with an enteric coating that increases the survival of the bacteriophage through the harsh environment in the stomach. The enteric coating is one which resists the action of gastric juices in the stomach so that the bacteriophages which are incorporated therein will pass through the stomach and into the intestines. The enteric coating may readily dissolve when in contact with intestinal fluids, so that the bacteriophage enclosed in the coating will be released in the intestinal tract. Enteric coatings may consist of polymer and copolymers well known in the art, such as commercially available EUDRAGIT (Evonik Industries). (See e.g., Zhang, AAPS PharmSciTech, (2016) 17 (1), 56-67).

According to a specific embodiment, the phage targeting *Bacteroides fragilis* or the pharmaceutical compositions comprising at least one phage targeting *Bacteroides fragilis* is administered orally and is associated with a proton pump inhibitor (PPI) such as, for example, omeprazole, lansoprazole, dexlansoprazole, esomeprazole, pantoprazole, rabeprazole and ilaprazole.

Dosages and desired drug concentrations of the pharmaceutical compositions may vary depending on the particular use. The determination of the appropriate dosage or route of administration is well within the skill of an ordinary physician. Animal experiments can provide reliable guidance for the determination of effective doses in human therapy. Interspecies scaling of effective doses can be performed by one of ordinary skill in the art following the principles described by Mordenti, J. and Chappell, W. (1989) Toxicokinetics and New Drug Development, Yacobi et al., Eds., Pergamon Press, New York 1989, pp42-96. Based on previous human experience in Europe, a dose of phage between 10⁷ and 10¹¹ PFU will be suitable in most instances (https://clinicaltrials.gov/ct2/show/NCT04737876,
https://doi.org/10.1016/j.cell.2022.07.003).

The phage targeting *Bacteroides fragilis* or the pharmaceutical compositions comprising the phage targeting *Bacteroides fragilis* may be administered as a single application, periodic applications, or as a continuous application.

According to another embodiment, the present invention relates to a probiotic composition comprising one or more isolated bacterial strains of species selected from the group consisting of *Bacteroides stercoris* and related phylotype *Bacteroides sp. DJF_B097* and/or *Prevotella copri* and related phylotype *Prevotella sp. DJF_RP53* for preventing cancer relapse in a patient having received an allogeneic hematopoietic stem cell transplantation (allo-HSCT).

In some embodiments, the bacterial strains are isolated. Any of the bacterial strains described herein may be isolated and/or purified, for example, from a source such as a culture or a microbiota sample (*e.g.,* fecal matter). The bacterial strains used in the compositions provided herein generally are isolated from the microbiome of healthy individuals. As also used herein, the term "purified" refers to a bacterial strain or composition comprising such that has been separated from one or more components, such as contaminants. In some embodiments, the bacterial strain is substantially free of contaminants. In some embodiments, one or more bacterial strains of a composition may be independently purified from one or more other bacteria produced and/or present in a culture or a sample containing the bacterial strain. In some embodiments, a bacterial strain is isolated or purified from a sample and then cultured under the appropriate conditions for bacterial replication, e.g., under anaerobic culture conditions. The bacteria that is grown under appropriate conditions for bacterial replication can subsequently be isolated/purified from the culture in which it is grown.

Any of the compositions described herein, including the probiotic compositions comprising the compositions, may contain bacterial strains in any form, for example in an aqueous form, such as a solution or a suspension, embedded in a semi-solid form, in a powdered form or freeze-dried form. In some embodiments, the composition or the bacterial strains of the composition are lyophilized. In some embodiments, a subset of the bacterial strains in a composition is lyophilized. Methods of lyophilizing compositions, specifically compositions comprising bacteria, are well known in the art. See, *e.g.,* US3,261,761; US4,205,132; PCT Publications WO2014/029578 and WO2012/098358, herein incorporated by reference in their entirety. The bacteria may be lyophilized as a combination and/or the bacteria may be lyophilized separately and combined prior to administration. A bacterial strain may be combined with a pharmaceutical excipient prior to combining it with the other bacterial strain or multiple lyophilized bacteria may be combined while in lyophilized form and the mixture of bacteria, once combined may be subsequently be combined with a pharmaceutical excipient. In some embodiments, the bacterial strain is a lyophilized cake. In some embodiments, the compositions comprising the one or more bacterial strains are a lyophilized cake.

Here again, said probiotic composition is formulated for delivery to the intestine (e.g., the small intestine and/or the colon).

In a preferred embodiment, the probiotic composition can be administered by in the form of a suppository or orally as detailed above.

According to another embodiment, the present invention relates to a nutritional composition comprising at least one metabolite involved in a metabolite subpathway selected in the group consisting of Fatty Acid Metabolism (also BCAA Metabolism); Phospholipid Metabolism; Lysophospholipid; Secondary Bile Acid Metabolism and Xanthine Metabolism.

Preferably, the metabolite is selected in the group consisting of:

| **metabolite** | **Superpathway** | **Subpathway** |
|---|---|---|
| deoxycholic acid 12-sulfate* | Lipid | Secondary Bile Acid Metabolism |
| deoxycholic acid 3-sulfate | Lipid | Secondary Bile Acid Metabolism |
| 1-methylxanthine | Xenobiotics | Xanthine Metabolism |
| deoxycholate | Lipid | Secondary Bile Acid Metabolism |
| propionylglycine (C3) | Lipid | Fatty Acid Metabolism (also BCAA Metabolism) |
| lithocholate | Lipid | Secondary Bile Acid Metabolism |
| 12-ketolithocholate | Lipid | Secondary Bile Acid Metabolism |
| lithocholic acid sulfate (2) | Lipid | Secondary Bile Acid Metabolism |
| hexadecasphinganine (d16:0)^{∗} | Lipid | Sphingolipid Synthesis |
| ursocholate | Lipid | Secondary Bile Acid Metabolism |
| taurocholenate sulfate^{∗} | Lipid | Secondary Bile Acid Metabolism |
| 3-dehydrodeoxycholate | Lipid | Secondary Bile Acid Metabolism |
| isoursodeoxycholate | Lipid | Secondary Bile Acid Metabolism |
| 1-stearoyl-GPI (18:0) | Lipid | Lysophospholipid |
| butyrylglycine (C4) | Lipid | Fatty Acid Metabolism (also BCAA Metabolism) |
| dehydrolithocholate | Lipid | Secondary Bile Acid Metabolism |
| 1-stearoyl-GPA (18:0) | Lipid | Lysophospholipid |
| glycodeoxycholate 3-sulfate | Lipid | Secondary Bile Acid Metabolism |
| lithocholate sulfate (1) | Lipid | Secondary Bile Acid Metabolism |
| 1-palmitoyl-GPG (16:0)^{∗} | Lipid | Lysophospholipid |
| 1-palmitoyl-GPI^{∗} (16:0) | Lipid | Lysophospholipid |

In a specific embodiment of the invention, the pharmaceutical compositions comprising the at least one phage targeting *Bacteroides fragilis,* the probiotic composition and the nutritional composition may be combined and administered for preventing cancer relapse in a patient having received an allogeneic hematopoietic stem cell transplantation (allo-HSCT).

### FIGURES LEGENDS

**Figure 1** - Gut bacteria characteristics following allogeneic hematopoietic stem cell transplant: Bar plots exhibiting top 10 absolute permutational multivariate analysis of variance (PERMANOVA) coefficient of phylotypes (here OTUs) in each enterotype.
**Figure 2** - Correlations between enterotypes and metabolite levels and viruses' frequency after allogeneic hematopoietic stem cell transplant: Dot plot summarizing enrichment factors (EF) and p-values of enriched subpathways from differentially detected metabolites levels. Enrichment factors were computed using over-representation method and P-value using hypergeometric test.
**Figure 3** - Principal coordinate analysis of viral reads and viral clusters composition: Principal coordinate analysis with UniFrac distance matrix from bacteriophages reads only. Samples were clustered with hierarchical Kmeans. Driving species were computed with permutational multivariate analysis of variance (PERMANOVA).
**Figure 4** - Correlations between enterotypes and metabolite levels and viruses' frequency after allogeneic hematopoietic stem cell transplant: Summarized correlation network illustrating subpathways and viral species associated with enterotype top driving phylotypes. For visualization purposes, phylotypes are described with the corresponding species names. Staph: *Staphylococcus;* Lact: *Lactococcus,* Mycobact: *Mycobacterium*
**Figure 5** - Viruses' genus associated with enterotypes top driving phylotypes: Tile plot showing number of species associated with phylotypes and which belong to the same genus.
**Figure 6** - Enterotype according groups of randomization and relapse at 12 months: Bar plot showing frequencies of samples in complete response or relapse at 12 months according to enterotypes.
**Figure 7** - Multivariate multinomial regression models evaluating enterotypes and clinical variable association.
   (A) Multivariate model evaluating relapse status
   (B) Multivariate model evaluating arm of treatment
   Multivariate model includes variables associated with enterotypes. Grey dots and line show statistically significant coefficients. Coefficients are relative to enterotype 1 and unmentioned group in each variable. GVHD: graft-versus-host disease, CsA: cyclosporine, MMF: mycophenolate mofetil, MTX: methotrexate, HSC: hematopoietic stem cell.

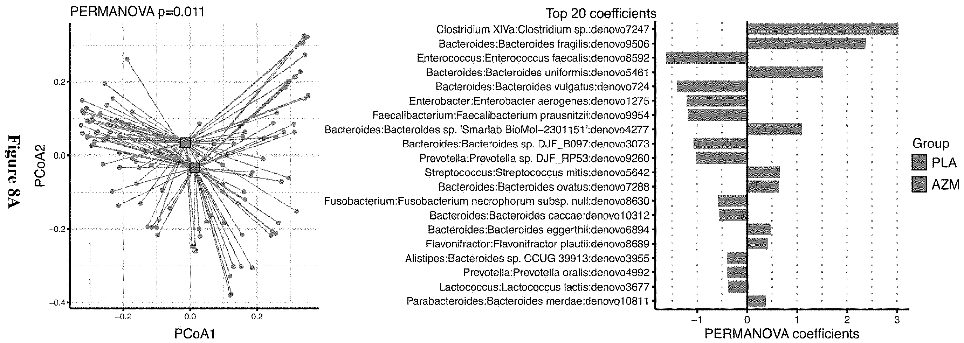

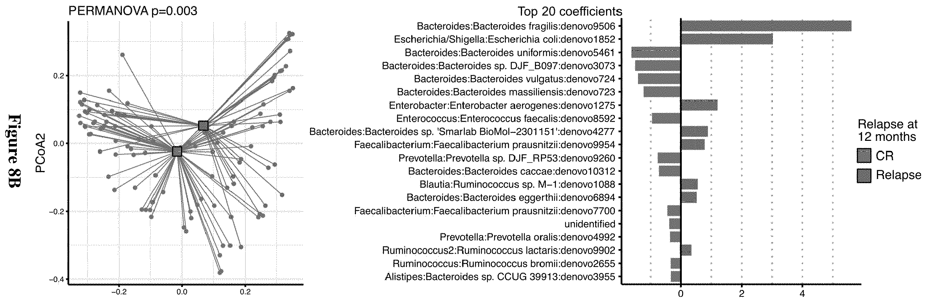

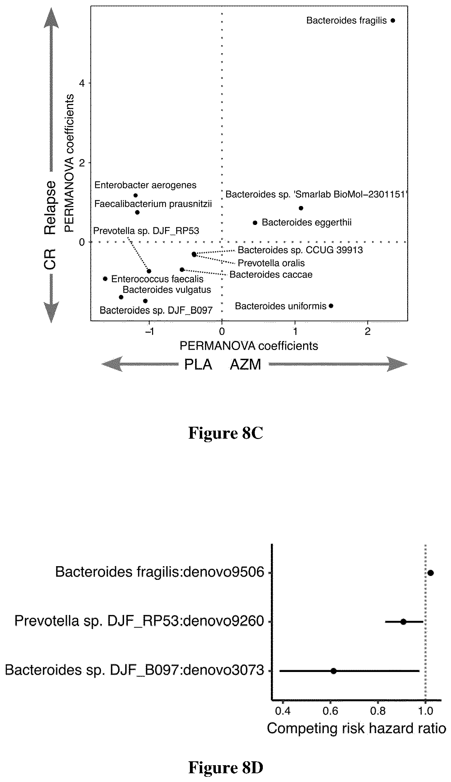

**Figure 8** **- Specific gut microbiota associated with azithromycin intake and relapse after transplant**
   (A-B) Dot plot depicting principal coordinate analysis (PCoA) computed from Bray-Curtis distance matrix. Squares show barycenter of each group. Bar plots represent top 20 phylotypes that drive the differences between the groups (PERMANOVA coefficients). Panel A compares patients from the azithromycin arm to those from placebo arm. Panel B compares patients in relapse at 12 months and those un complete response.
   (C) Dot plot showing PERMANOVA coefficients of phylotype found in the top 20 coefficients in both relapse/complete response and azithromycin/placebo enterotypes.
   (D) Forest plot showing hazard ratio and 95% confidence intervals of relapse according to Fine and Gray competing risk model of relapse with death as a competing risk. The analysis was performed with phylotype shown in panel C, for visualization purposes, only significant phylotypes are shown.

### EXAMPLES

### I. Materials and Methods

### Cohort

Fecal samples from patients included in the multicenter, randomized, double-blind, placebo-controlled phase 3 superiority trial ALLOZITHRO ((NCT01959100) were collected overtime, including 27 patients from the azithromycin arm (n=73 samples) and 28 patients from the placebo arm (n=75 samples). First and last samples were respectively collected from the week before allo-HSCT to 6 weeks after allo-HSCT. Most samples were assessed for all omics (n=92), including 43 and 49 in the placebo and azithromycin cohorts, respectively. Characteristics of patients included in placebo and azithromycin cohort were similar. Nutrition support and concomitant antibiotics used were also similar in both arms.

### Bacteriome

Sample processing and 16SrRNA gene sequencing. Patients' fecal samples were frozen-aliquoted (150 mg), homogenized and lyzed using both mechanical (beadbeating for 10 minutes) and chemical techniques as previously described (Doi: 10.1038/nature08821), and total DNA was extracted using the MoBio Power Fecal DNA isolation kit following the manufacturer's recommendations. DNA quality and quantity were evaluated using a spectrophotometer (Nanodrop 1000, Thermoscientific). Sequencing was then performed at GeT-PlaGe platform of the Génopole (Toulouse Midi-Pyrénées, France) using Illumina MiSeq technology targeting the V3-V4 region of the 16SrRNA gene with the following primers: V3fwd-TACGGRAGGCAGCAG wherein R is A or G and V4rev-TACCAGGGTATCTAAT.

Phylotypes identification pipeline. The raw sequences were analyzed using the open source software package Quantitative Insights Into Microbial Ecology (QIIME) (Caporaso et al. (2010) Nat Methods 7, 335-336). After trimming primers and barcodes, the sequences were filtered for quality (minimum length= 300bp, minimum quality threshold=20, chimeras removal) and clustered into operational taxonomic units (OTU) at a threshold of 97% similarity level using uclust. Samples amplified but resulting in less than 500 reads were removed. The most abundant member of each OTU was selected as the representative sequence and assigned to different taxonomic levels using the RDP naive Bayesian classifier and RDP Seqmatch program (Cole et al. (2009) Nucleic Acids Res 37, D141-14). Estimates of phylotypes richness and diversity were calculated using the number of observed OTUs, Shannon and Simpson indices on the rarefied OTU table (n=3,000 reads). An average of 14,677 reads were obtained per sample (ranging from 3,056 to 24,545).

### Virome

DNA and RNA virome analysis with shotgun Next Generation Sequencing. Fecal samples (solid phase) were re-suspended and diluted (50%) in phosphate buffered saline (PBS) and then centrifuged at 2500 g for 20 minutes. To enrich for viral particles by reduction of host background, stool supernatant was filtered through a 0.45 µm filter (Corning Costar Spin-X centrifuge tube filters), and an aliquot of 315 µl of filtrate was pretreated before extraction by incubation with different nucleases: TURBO DNAse (Invitrogen, Carlsbad, CA); Baseline-ZERO DNase (Ambion, Foster City, CA); Benzonase (NEB); RNAse A (Promega) for 30 min, at 37°C. Total nucleic acids were extracted using NucliSENS easyMAG (Biomerieux) according manufacturers protocol. For DNA libraries preparation, 25 µL of extract was used. Depletion of methylated host DNA was performed using NEBNext ^{®} Microbiome DNA Enrichment Kit (NEB) according to the manufacturer instructions. DNA was then purified using zymo DNA Clean (Zymo) and eluted in 7.5 µL of sterile water. DNA libraries were prepared using Nextera XT library preparation kit (Illumina). For RNA libraries preparation, Trio RNA Kit (TECAN) was used according to manufacturer instructions. Libraries were sequenced on an Illumina HiSeq X (16 lanes) using 150/150-bp paired-end sequencing.

Species identification pipeline. Raw reads were cleaned using TRIMMOMATIC (Bolger, A.M., Lohse, M., and Usadel, B. (2014) Bioinformatics 30, 2114-2120). Duplicated reads were removed using Dedupe (Gregg, F., and Eder, D. (2022). Dedupe. https://github.com/dedupeio/dedupe). Taxonomic assignment was carried out using Kraken2 with Viral, Bacterial and Human Refseq databases (Wood, D.E., Lu, J., and Langmead, B. (2019) Genome Biol 20, 257). Kraken viral assigned reads were verified using Blastn on Refseq viral database. Reads with inconsistent assignment between Kraken and Blast methods were removed. Samples with less than 5.10⁶ reads were excluded. Data with less than 0.5 read per million (RPM) were assimilated to 0. Variables were filtered according to mean RPM in seven negative controls (Miller et al. (2019) Genome Res. 29, 831-842). DNA and RNA reads databases, were then merged to obtain a final count database.

### Fecal metabolomics

Sample processing. Samples were processed and analyzed by Ultrahigh Performance Liquid Chromatography-Tandem Mass Spectroscopy (UPLC-MS/MS) by Metabolon (Durham, USA). Acquisition, quality control and metabolites identification and quantification were performed as previously described (Michonneau, D. (2019). Nature Communications, 10, 5695).

Pathway identification. To assign metabolic pathways, the list of identified metabolites was manually compared to Metabolon, Human Metabolome Database (Wishart et al. (2007) HMDB: the Human Metabolome Database. Nucleic Acids Research 35, D521-D526) and PubChem databases (Kim et al. (2021). PubChem in 2021: new data content and improved web interfaces. Nucleic Acids Research 49).

Metabolite data preprocessing. Uncharacterized metabolites were excluded from the analyses. Quantification of metabolites was normalized to dry weight of fecal material extracted. Missing values (metabolites below quantification threshold) were imputed with 50% of the minimum value of the corresponding metabolite, and metabolites with more than 50% of missing values were excluded. Regarding drugs-related metabolites, missing values were imputed with 1% of the minimum value and none of the metabolites were excluded.

### Data and statistical analyses

Enterotype definition. 'vegan' package was used to compute dissimilarity matrix with Bray-Curtis and UniFrac methods for bacterial and viral data, respectively (Oksanen *et al.* R. vegan: Community Ecology Package. R package version 2.6-2. https://CRAN.R-project.org/package=vegan). Dimension reduction of dissimilarity matrix was done with principal coordinate analysis (PCoA). Sample clustering was performed with hierarchical Kmeans with the package 'factoextra' (Kassambara, A., and Mundt, F. Extract and Visualize the Results of Multivariate Data Analyses. R package. version 1.0.7. https://CRAN.R-project.org/package=factoextra). To identify leading phylotype in the clustering we used permutational multivariate analysis of variance (PERMANOVA) with 999 permutations.

Correlation analysis. Using non-parametric Spearman's method, a correlation matrix was computed with every variable from the three omics datasets. To keep relevant correlation, only these with an absolute rho coefficient above 0.3 and statistically significant (false discovery rate adjusted p-value < 0.05) were kept.

Correspondence analysis. Correspondence analysis was performed with "FactoMineR" package (Lê, S., Josse, J., and Husson, F. (2008). J. Stat. Soft. 25.), with all antibiotics used during allo-HSCT procedure.

Metabolomic analyses. Pathway enrichment was evaluated with enrichment factor computed with over-representation analysis method. Statistical testing to enrichment was done with hypergeometric distribution (Michonneau, 2019).

Network analysis. Network were built from correlation matrices. Each node shows one variable and edges depict significant correlations. In metabolic enriched pathway analyses, edges depict a link between the pathway and the OTU. For visualization purposed Fruchterman-Reingold algorithm was used.

Network modules analysis. Modules were defined after Louvain clustering applied to build networks. Principal component analysis (PCA) was used to calculate contribution and loadings of each variable into the module. Variables included in one module are included in a PCA. Then, loadings are computed with variables coordinates on the first PCA axis. Then, for each sample, the modules variables have been computed by summing individual variables weighted with the loadings.

Survival analyses. Incidence of relapse was computed with competing risk model using Fine and Gray method. Starting point was the day of infused graft (D0), death was considered as a competing risk event and relapse the event. Mean value was applied in case of multiple samples from one patient.

Statistical tests. Considering that variables distribution is non-gaussian, bilateral, non-parametric tests were used. To study enterotypes association with feces and plasma metabolites, viruses and T cells subsets, non-parametric Kruskall-Wallis test was used. Frequency comparisons were performed with chi-2 or Fisher test. False discovery rate method was used to correct P-value for multiple testing with Benjamini-Hochberg method. Multinomial regression was used to build models to test multivariate association between enterotypes and clinical variables. Linear regression models were built to test multivariate association between modules and clinical variables. To evaluate phylotypes associations with T cells subsets, linear regression was used: mean phylotype frequency association with T cells subsets was evaluated.

### Software

Computational environment may be reproduced using GNU Guix (guix.gnu.org) with the files 'manifest.scm' and 'channels.scm' in 'guixconfig' directory of the git repository (Vallet, N., Michonneau, D., and Tournier, S. (2022). Toward practical transparent verifiable and long-term reproducible research using Guix. Sci Data 9, 597).

### II. Results

### Post-transplant gut microbial is characterized by four enterotypes

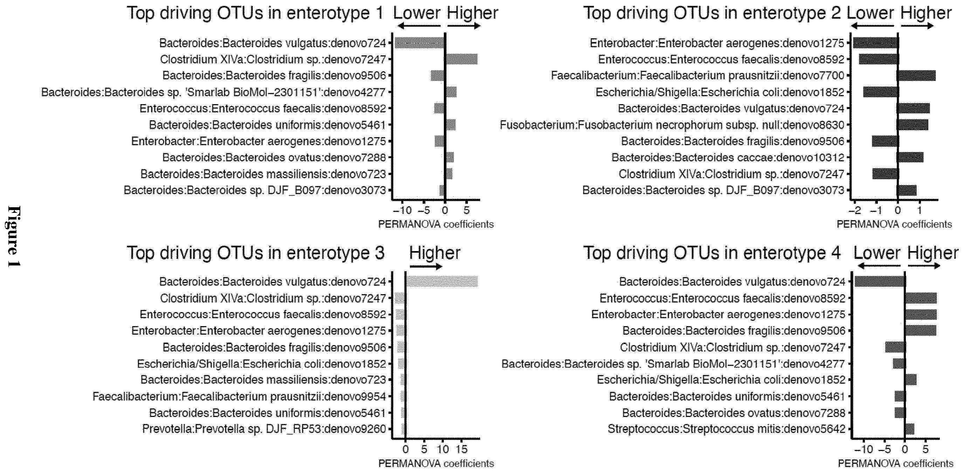

First, the post-transplant gut microbiota has been characterizing. Microbial load was stable overtime, α-diversity measured with number of OTUs, Simpson and Shannon indices decreased during the procedure, mainly during the two first weeks after transplantation. It has next been evaluated whether samples would cluster in enterotypes. Using k-means clustering, four clusters of samples have been uncovered. These clusters were associated with α-diversity: cluster 2 exhibited the highest diversity, followed by cluster 1, 3 and 4. Each cluster was characterized by specific bacterial genera and phylotypes abundances. Enterotype 1 was characterized by higher proportions of *Clostridium sp.* and lower *Bacteroides vulgatus.* Enterotype 2 was characterized by higher proportions of *Faecalibacterium prausnitzii, B. vulgatus, Fusobacterium necrophorum* and *Bacteroides caccae* and lower *Enterobacter aerogenes, Enterococcus faecalis, Escherichia coli, Bacteroides fragilis* and Clostridium sp. Enterotype 3 was mainly driven by *B. vulgatus* while enterotype 4 was enriched in *E. faecalis, E. aerogenes,* and *B. fragilis* (**Figure 1**).

### Post-transplant gut bacteriome is associated with specific metabolic pathways and bacteriophages populations

To explore microbiome functions, the gut metabolome has been analyzed and 925 known feces metabolites have been detected. Among these metabolites, 99 were significantly associated with the four enterotypes distribution (**Table 1**).

**Table 1**

| **metabolite** | **Superpathway** | **Subpathway** | **pval** | **fdrpval** |
|---|---|---|---|---|
| 3-(4-hydroxyphenyl)propionate | Xenobiotics | Benzoate Metabolism | 1,60E-06 | 0,00147484 |
| N-propionylalanine | Amino Acid | Alanine and Aspartate Metabolism | 3,68E-06 | 0,00152717 |
| 5-hydroxypicolinic acid | Amino Acid | Tryptophan Metabolism | 4,96E-06 | 0,00152717 |
| beta-citrylglutamate | Amino Acid | Glutamate Metabolism | 3,82E-05 | 0,00783037 |
| 2-acetamidobutanoate | Xenobiotics | Bacterial/Fungal | 4,24E-05 | 0,00783037 |
| (12 or 13)-methylmyristate (a15:0 or i15:0) | Lipid | Fatty Acid, Branched | 5,28E-05 | 0,00812281 |
| propionylglutamine | Amino Acid | Glutamate Metabolism | 8,90E-05 | 0,00962663 |
| solanidine | Xenobiotics | Food Component/Plant | 0,0001039 | 0,00962663 |
| N-butyryl-leucine | Amino Acid | Leucine, Isoleucine and Valine Metabolism | 0,00010574 | 0,00962663 |
| N-palmitoyl-phytosphingosine (t18:0/16:0) | Lipid | Ceramides | 0,00011458 | 0,00962663 |
| deoxycholic acid 12-sulfate* | Lipid | Secondary Bile Acid Metabolism | 0,00011473 | 0,00962663 |
| deoxycholic acid 3-sulfate | Lipid | Secondary Bile Acid Metabolism | 0,00014742 | 0,01110966 |
| 1-methylxanthine | Xenobiotics | Xanthine Metabolism | 0,00016193 | 0,01110966 |
| 5,6-dihydrothymine | Nucleotide | Pyrimidine Metabolism, Thymine containing | 0,00021342 | 0,01110966 |
| hypoxanthine | Nucleotide | Purine Metabolism, (Hypo)Xanthine/in osine containing | 0,00022965 | 0,01110966 |
| deoxycholate | Lipid | Secondary Bile Acid Metabolism | 0,00023023 | 0,01110966 |
| (R)-salsolinol | Amino Acid | Tyrosine Metabolism | 0,00023205 | 0,01110966 |
| 3-hydroxymargarate | Lipid | Fatty Acid, Monohydroxy | 0,00023435 | 0,01110966 |
| butyrylglutamine/isobutyry Iglutamine | Amino Acid | Glutamate Metabolism | 0,00023941 | 0,01110966 |
| undecanedioate (C11-DC) | Lipid | Fatty Acid, Dicarboxylate | 0,00024073 | 0,01110966 |
| norvaline | Amino Acid | Urea cycle; Arginine and Proline Metabolism | 0,00025638 | 0,01126829 |
| N-butyryl-phenylalanine | Amino Acid | Phenylalanine Metabolism | 0,00027956 | 0,01164669 |
| N-butyryl-isoleucine | Amino Acid | Leucine, Isoleucine and Valine Metabolism | 0,00029022 | 0,01164669 |
| N-methylalanine | Amino Acid | Alanine and Aspartate Metabolism | 0,00035038 | 0,0130763 |
| N-propionylmethionine | Amino Acid | Methionine, Cysteine, SAM and Taurine Metabolism | 0,00036235 | 0,0130763 |
| propionylglycine (C3) | Lipid | Fatty Acid Metabolism (also BCAA Metabolism) | 0,00037854 | 0,0130763 |
| thymine | Nucleotide | Pyrimidine Metabolism, Thymine containing | 0,00039306 | 0,0130763 |
| hexadecanedioate (C16) | Lipid | Fatty Acid, Dicarboxylate | 0,00039668 | 0,0130763 |
| 3-(3-hydroxyphenyl)propionate | Xenobiotics | Benzoate Metabolism | 0,00041651 | 0,01325638 |
| lithocholate | Lipid | Secondary Bile Acid Metabolism | 0,000495 | 0,01522939 |
| arachidoylcarnitine (C20)^{∗} | Lipid | Fatty Acid Metabolism (Acyl Carnitine, Long Chain Saturated) | 0,00055326 | 0,01631307 |
| uracil | Nucleotide | Pyrimidine Metabolism, Uracil containing | 0,00056557 | 0,01631307 |
| 3,4-dihydroxyphenylacetate | Amino Acid | Tyrosine Metabolism | 0,00064316 | 0,01798893 |
| beta-alanine | Nucleotide | Pyrimidine Metabolism, Uracil containing | 0,0006838 | 0,01856319 |
| 2-piperidinone | Xenobiotics | Food Component/Plant | 0,00072392 | 0,01863683 |
| erucoylcarnitine (C22:1)* | Lipid | Fatty Acid Metabolism (Acyl Carnitine, Monounsaturated) | 0,0007269 | 0,01863683 |
| 12-ketolithocholate | Lipid | Secondary Bile Acid Metabolism | 0,00082599 | 0,01944465 |
| lithocholic acid sulfate (2) | Lipid | Secondary Bile Acid Metabolism | 0,00082685 | 0,01944465 |
| hexadecasphinganine (d16:0)^{∗} | Lipid | Sphingolipid Synthesis | 0,00083427 | 0,01944465 |
| ursocholate | Lipid | Secondary Bile Acid Metabolism | 0,00085515 | 0,01944465 |
| betaine | Amino Acid | Glycine, Serine and Threonine Metabolism | 0,00086374 | 0,01944465 |
| N-acetylhomocitrulline | Amino Acid | Urea cycle; Arginine and Proline Metabolism | 0,00091529 | 0,01981488 |
| valylglutamine | Peptide | Dipeptide | 0,00092312 | 0,01981488 |
| N-acetylglucosamine/N-acetylgalactosamine | Carbohydrate | Aminosugar Metabolism | 0,0009602 | 0,02014233 |
| riboflavin (Vitamin B2) | Cofactors and Vitamins | Riboflavin Metabolism | 0,00102249 | 0,02060344 |
| sphinganine | Lipid | Sphingolipid Synthesis | 0,00102682 | 0,02060344 |
| taurocholenate sulfate^{∗} | Lipid | Secondary Bile Acid Metabolism | 0,00105504 | 0,0207191 |
| glycochenodeoxycholate glucuronide (1) | Lipid | Primary Bile Acid Metabolism | 0,00121573 | 0,02337755 |
| octadecenedioate (C18:1-DC) | Lipid | Fatty Acid, Dicarboxylate | 0,00129422 | 0,02437885 |
| 10-hydroxystearate | Lipid | Fatty Acid, Monohydroxy | 0,00133325 | 0,02461182 |
| nicotinate | Cofactors and Vitamins | Nicotinate and Nicotinamide Metabolism | 0,00163205 | 0,02953688 |
| 3-dehydrodeoxycholate | Lipid | Secondary Bile Acid Metabolism | 0,00177624 | 0,0315165 |
| valerylphenylalanine | Amino Acid | Phenylalanine Metabolism | 0,00180972 | 0,0315165 |
| ribose | Carbohydrate | Pentose Metabolism | 0,00191685 | 0,03245045 |
| glycocholate | Lipid | Primary Bile Acid Metabolism | 0,00197487 | 0,03245045 |
| 3-hydroxymargaroylglycine | Lipid | Fatty Acid Metabolism (Acyl Glycine) | 0,00200111 | 0,03245045 |
| isoursodeoxycholate | Lipid | Secondary Bile Acid Metabolism | 0,00206777 | 0,03245045 |
| D-urobilin | Cofactors and Vitamins | Hemoglobin and Porphyrin Metabolism | 0,00212452 | 0,03245045 |
| N-carbamoylputrescine | Amino Acid | Polyamine Metabolism | 0,00214266 | 0,03245045 |
| 2-palmitoyl-galactosylglycerol (16:0)^{∗} | Lipid | Galactosyl Glycerolipids | 0,00214817 | 0,03245045 |
| tricarballylate | Energy | TCA Cycle | 0,00215057 | 0,03245045 |
| 1-stearoyl-GPI (18:0) | Lipid | Lysophospholipid | 0,00217977 | 0,03245045 |
| indolin-2-one | Xenobiotics | Food Component/Plant | 0,00231425 | 0,03390564 |
| butyrylglycine (C4) | Lipid | Fatty Acid Metabolism (also BCAA Metabolism) | 0,00257893 | 0,03719295 |
| methionine sulfoxide | Amino Acid | Methionine, Cysteine, SAM and Taurine Metabolism | 0,00266475 | 0,03744234 |
| 1-ribosyl-imidazoleacetate^{∗} | Amino Acid | Histidine Metabolism | 0,00267735 | 0,03744234 |
| cysteine sulfinic acid | Amino Acid | Methionine, Cysteine, SAM and Taurine Metabolism | 0,00276208 | 0,03805074 |
| glycochenodeoxycholate | Lipid | Primary Bile Acid Metabolism | 0,00292767 | 0,03973884 |
| dehydrolithocholate | Lipid | Secondary Bile Acid Metabolism | 0,00302517 | 0,0404671 |
| citraconate/glutaconate | Energy | TCA Cycle | 0,00318162 | 0,04135023 |
| allo-threonine | Amino Acid | Glycine, Serine and Threonine Metabolism | 0,0032239 | 0,04135023 |
| 3-hydroxypalmitate | Lipid | Fatty Acid, Monohydroxy | 0,00322559 | 0,04135023 |
| isobutyrylglycine (C4) | Amino Acid | Leucine, Isoleucine and Valine Metabolism | 0,00334952 | 0,04235071 |
| N-linoleoyltaurine^{∗} | Lipid | Endocannabinoid | 0,00352029 | 0,04390848 |
| gentisate | Amino Acid | Tyrosine Metabolism | 0,00376103 | 0,04579147 |
| sulfamethoxazole | Xenobiotics | Drug - Antibiotic | 0,00377048 | 0,04579147 |
| 1-stearoyl-GPA (18:0) | Lipid | Lysophospholipid | 0,00395526 | 0,04688837 |
| methylsuccinate | Amino Acid | Leucine, Isoleucine and Valine Metabolism | 0,0039624 | 0,04688837 |
| eicosenoylcarnitine (C20:1)^{∗} | Lipid | Fatty Acid Metabolism (Acyl Carnitine, Monounsaturated) | 0,00406351 | 0,04747622 |
| glycodeoxycholate 3-sulfate | Lipid | Secondary Bile Acid Metabolism | 0,00424717 | 0,04812396 |
| androsterone sulfate | Lipid | Androgenic Steroids | 0,00430078 | 0,04812396 |
| N-formylanthranilic acid | Amino Acid | Tryptophan Metabolism | 0,00430179 | 0,04812396 |
| lithocholate sulfate (1) | Lipid | Secondary Bile Acid Metabolism | 0,00432751 | 0,04812396 |
| 1-methylguanosine | Nucleotide | Purine Metabolism, Guanine containing | 0,00455219 | 0,04950308 |
| 1-palmitoyl-GPG (16:0)^{∗} | Lipid | Lysophospholipid | 0,00465171 | 0,04950308 |
| S-carboxymethyl-L-cysteine | Xenobiotics | Drug - Other | 0,00474783 | 0,04950308 |
| 3-hydroxyhexanoate | Lipid | Fatty Acid, Monohydroxy | 0,00477195 | 0,04950308 |
| 3-phenylpropionate (hydrocinnamate) | Xenobiotics | Benzoate Metabolism | 0,00480352 | 0,04950308 |
| 3-hydroxystearate | Lipid | Fatty Acid, Monohydroxy | 0,00481443 | 0,04950308 |
| quinolinate | Cofactors and Vitamins | Nicotinate and Nicotinamide Metabolism | 0,00484372 | 0,04950308 |
| N-acetylglucosaminylasparagi ne | Carbohydrate | Aminosugar Metabolism | 0,00495067 | 0,04950308 |
| N-methylleucine | Amino Acid | Leucine, Isoleucine and Valine Metabolism | 0,00498726 | 0,04950308 |
| imidazole propionate | Amino Acid | Histidine Metabolism | 0,00498785 | 0,04950308 |
| stearoylcarnitine (C18) | Lipid | Fatty Acid Metabolism (Acyl Carnitine, Long Chain Saturated) | 0,00504837 | 0,04957067 |
| N-methylproline | Amino Acid | Urea cycle; Arginine and Proline Metabolism | 0,00517321 | 0,049838 |
| chenodeoxycholic acid sulfate (1) | Lipid | Primary Bile Acid Metabolism | 0,00521354 | 0,049838 |
| pipecolate | Amino Acid | Lysine Metabolism | 0,00529467 | 0,049838 |
| glutarate (C5-DC) | Lipid | Fatty Acid, Dicarboxylate | 0,00533002 | 0,049838 |
| 1-palmitoyl-GPI^{∗} (16:0) | Lipid | Lysophospholipid | 0,00534557 | 0,049838 |

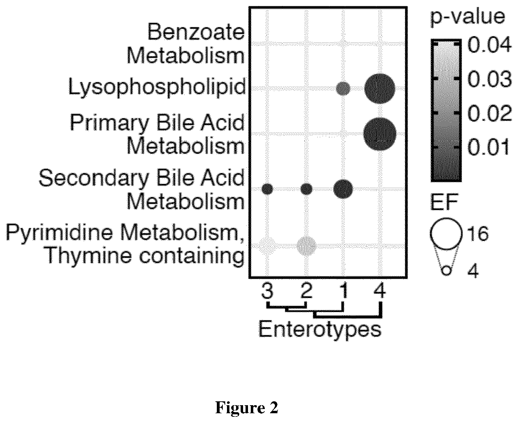

Enrichment analysis using 0 to 1 normalized value above 0.6 to define enriched metabolites, revealed specific metabolic profiles between enterotypes **(****Figure 2**). All enterotypes except number 4 were associated with secondary bile acid metabolism. Enterotype 4 exhibited enrichment of primary bile acid and lysophospholipids metabolism. Enterotype 1 was enriched in lysophospholipids and secondary bile acid metabolism. Enterotypes 2 and 3 were close and characterized by enrichment in metabolites involved in secondary bile acid metabolism and pyrimidine metabolism metabolites (**Figure 2**). *Bacteriophages species and feces metabolome are specifically associated with phylotypes enriched in enterotypes*

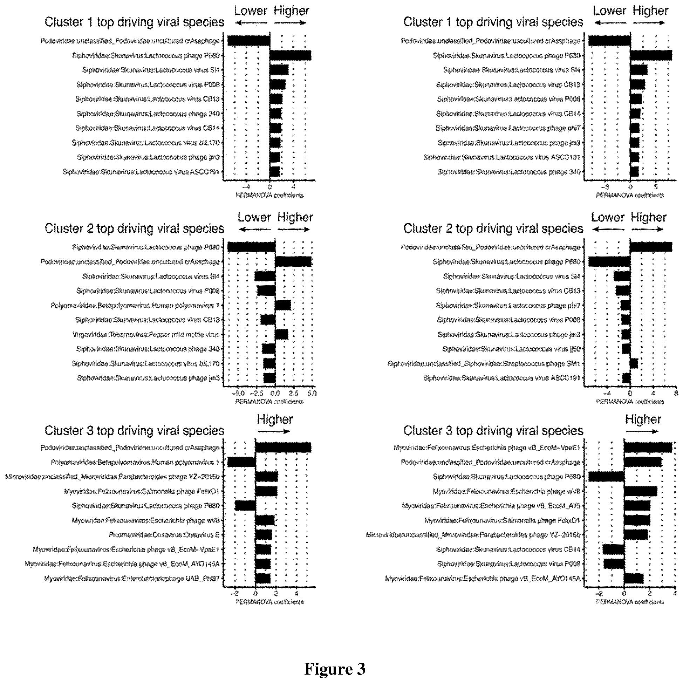

Then, to investigate how gut virome could impact gut microbiota homeostasis after allo-HSCT, viral species in feces samples have been studied. Individual viruses were not associated with bacterial enterotypes. To evaluate whether groups of viruses may be associated with bacterial enterotypes, samples were clustered with k-means method with either all viral species or with bacteriophages species. Clusters from all viruses reads were mainly driven by bacteriophages-restricted reads (**Figure 3**). These viral clusters were not associated with enterotypes.

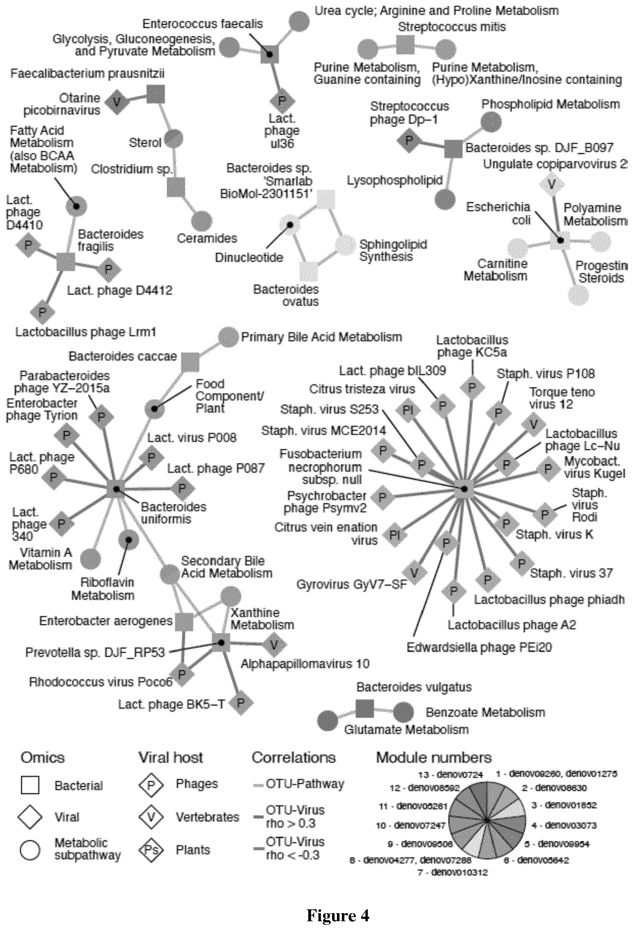

To study which phylotypes drove metabolic profiles and whether these may be influenced by gut virome, correlation analysis with the top 13 enterotype-driving phylotypes, the 99 metabolites associated with enterotypes and all viral species have been performed. This allowed the identification of 270 statistically significant correlations defined by absolute Spearman's rho above 0.3 and adjusted p-value below 0.05. These correlations were represented as a correlation network and Louvain clustering was applied to identify modules of correlated variables. Next, metabolites associated with phylotypes have been summarized into enriched metabolic pathways (**Figure 4**). Secondary bile acid enrichment was shared by *B. uniformis* (module 11), *E. aerogenes* and *Prevotella sp. DJF_RP53* (module 1). Sterol pathway enrichment was shared by *Clostridium sp.* (module 10) and *F*. *prausnitzii* (module 5).

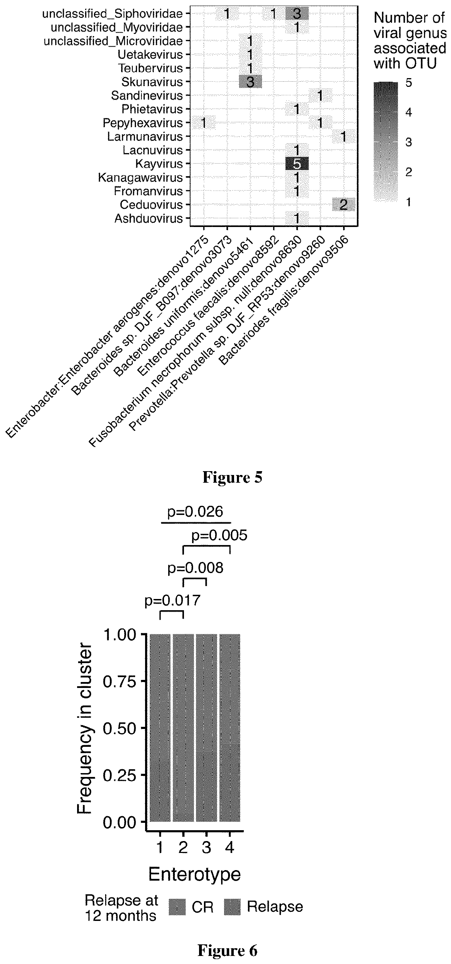

Among the 13 modules, the phylotypes related to *Fusobacterium necrophorum* (module 2) was associated with 18 viral species including 14 (78%) bacteriophages. When studying bacteriophages according to their genus, only *Pepyhexaviruses* were commonly associated with phylotypes related to *Prevotella sp. DJF_RP53* and *E. aerogenes.* Correlations with unclassified *Siphoviridae* were shared by phylotypes related to *Bacteroides sp. DJF-B097, E. faecalis* and *F. necrophorum* (**Figure 5**). Among eukaryotic host viruses, a species of *picobirnaviruses, Otarine picobirnavirus* was associated with a phylotype related to *F. prausnitzii* (module 5).

### Enterotypes are associated with azithromycin and subsequent relapse

It has then been evaluated whether clinical data and outcomes were associated with enterotypes. Belonging to one enterotype was fluctuating overtime for some patients. Among the 38 patients with at least 2 samples, 22 (58%) had a change of enterotype during the allo-HSCT procedure. Compared to those with stable enterotypes, patients who changed of enterotypes during transplantation procedure were associated with less relapse (complete remission at 12 months, n=17/22, 77% versus n=6/16, 38%, p=0.020). Shift in enterotype was not influenced by clinical variables nor by type of antibiotics used. Enterotypes were not influenced by time since allo-HSCT.

It has next been evaluated whether enterotypes and multi-omics modules were associated with clinical variables, notably azithromycin intake and relapse. ALLOZITHRO treatment arm (azithromycin or placebo) was associated with enterotypes (p=0.037). Enterotypes 3 and 4 were equally distributed in azithromycin and placebo samples, while enterotype 1 was associated with 23 (70%) samples from the azithromycin group and 17 (68%) samples from the placebo group were observed in enterotype 2 (p=0.02). Post-transplantation relapse of the underlying malignant disease at 12 months was associated with enterotypes distribution (p=0.026). Enterotype 2 was mainly associated with complete remission (95.5%) relapse while enterotypes 1, 3 and 4 were respectively associated with 10 (32%, p=0.017), 12 (38%, p=0.008) and 10 (42%, p=0.005) samples from relapsing patients (**Figure 6**). Among other clinical variables, type of donor (p=0.046), source of stem cells (p=0.044) and GVHD prophylaxis (p=0.038) were also associated with enterotypes. Acute and chronic GVHD were not associated with enterotypes.

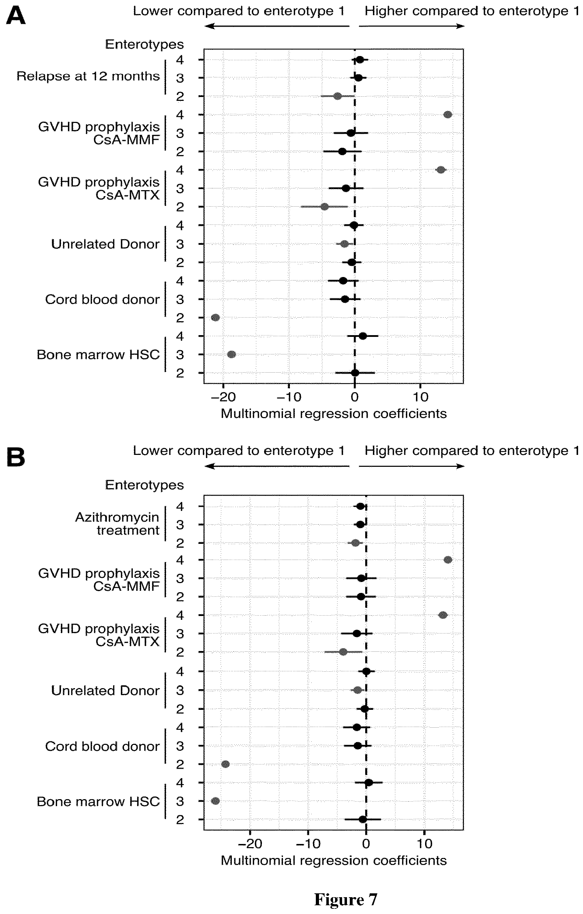

To avoid possible inference of confounding factors, a multivariate multinomial regression models using clinical variables associated with enterotypes as covariates has been performed. Relapse and azithromycin intake were still associated with enterotypes (**Figure 7**).

Type of nutrition support was not associated with enterotypes (p=0.092).

### Multi-omics modules are specifically associated with azithromycin intake and relapse

Since azithromycin (or placebo) intake and complete remission (or hematological relapse) were associated with enterotypes, it has been further explored if these parameters were associated within multi-omics modules. This univariate analysis revealed that azithromycin group was associated with lower module 5 and higher module 7 derived-variable. Relapse and complete remission were not associated with modules.

Multivariate analysis using variables associated with enterotypes as covariate (i) confirmed an association of module 5 and 7 with azithromycin and (ii) revealed module 12 as significantly associated with azithromycin intake. Module 5 included *F. prausnitzii, otariine picobirnavirus,* sterol, primary and secondary bile acid and fatty acid metabolites. Module 7 was driven by *B. caccae.* The latter module was enriched for primary bile acid metabolites and coffee-derived metabolites. Module 12 included *E. faecalis* which was inversely correlated with *Lactococcus phage ul36.*

In addition, module 9 was associated with relapse in multivariate analysis. Module 9 was centered by *B. fragilis* and correlated with *Lactobacillus phage Lrm1* while *Lactococcus phages D4412* and *D4410* and two metabolites from dicarboxylate and branched chain amino acids (BCAA) pathways were inversely correlated.

### Azithromycin and relapse share gut microbiota alterations

A supervised analysis was next applied to better characterize gut microbiota composition associated with azithromycin and relapse. Azithromycin and placebo intake were associated with a specific gut bacteriome (permutational multivariate analysis of variance, PERMANOVA p=0.011). Among the top driving phylotypes, those related to *Clostridium sp, B. fragilis, B. uniformis, Bacteroides sp. `Smarlab Biomol-2301151* ' were associated with azithromycin while *E. faecalis, B. vulgatus, E. aerogenes, F. prausnitzii, Bacteroides sp. DJF_B097* and *Prevotella sp. DJF_RP53* were associated with placebo (**Figure 8A**). Likewise, complete remission and relapses were associated with specific microbiota (PERMANOVA p=0.003). Relapse-associated microbiota was mainly characterized by phylotypes related to *B. fragilis, E. coli, E. aerogenes,* whereas remission was associated with OTUs related to *B. uniformis, Bacteroides sp. DJF_B097, B. vulgatus, B. massiliensis* and *E. faecalis* (**Figure 8B**).

PERMANOVA coefficients of randomization groups (placebo or azithromycin) and disease status (relapse or complete response) were crossed to highlight which phylotypes were associated with both variables (**Figure 8C**). Consistently with the higher risk of relapse in azithromycin group in the ALLOZITHRO trial, among the 13 phylotypes, 10 (77%) were associated with relapse and azithromycin or placebo and complete remission. Briefly, among the 10 phylotypes, relatives of *B. fragilis, Bacteroides sp. 'Smarlab BioMol-2301151*' and *B. eggerthii* were associated with relapse and azithromycin whereas *B. vulgatus, Bacteroides sp. DJF_B097, E. faecalis, Prevotella sp. DJF_RP53, B. caccae, P. oralis, Bacteroides sp. CCUG 39913* were associated with placebo and complete remission.

To evaluate which phylotypes were associated with relapse after taking into account the time-dependent nature of this outcome and death as a competing risk, a Fine and Gray model was computed. Mean was applied to phylotypes frequencies of patients with multiple samples. Results revealed that *B. fragilis* was associated with higher risk of relapse (HR=1.02, 95% confidence interval (95CI): 1.02-1.03, p<0.001). Two other phylotypes were associated with lower risk of relapse: *Prevotella sp. DJF_RP53* (HR=0.90, 95CI: 0.85-0.94, p<0.001), *Bacteroides sp. DJF_B097* (HR=0.69, 95CI: 0.57-0.82, p<0.001) (**Figure 8D**)**.**

The overtime frequency profiles overtime of the 3 phylotypes were comparable between complete remission and placebo, or azithromycin and relapsing patients. Phylotype related to *Bacteroides sp. DJF_B097* was lower in azithromycin and relapsing patient. *Prevotella sp. DJF_RP53* profiles of relapsed/azithromycin groups were inversely correlated with the ones from complete remission/placebo groups. By contrast, *B. fragilis* profiles were consistent with higher frequencies in azithromycin and relapsing patients. None of the phylotypes correlated together, suggesting that their trajectories were independent. Altogether, these results show that azithromycin intake impacts gut microbiota. *Bacteroides sp. DJF_B097, Prevotella sp. DJF_RP53* were associated with complete responses while *B. fragilis* was associated with higher risk of relapse.

### III. Conclusion

Early administration of azithromycin during allo-HSCT unexpectedly increased risk of hematological malignancies relapses in a randomized clinical trial (Bergeron *et al.* (2017) JAMA 318, 557). Here, by using unsupervised and targeted approaches, it has been revealed the impact of azithromycin treatment on gut microbiota which contributed to relapse.

Specific differences of gut microbiota from patients treated with azithromycin or placebo and from those how relapsed or remained in complete remission have been characterized. Among the overlapping phylotypes associated with azithromycin, placebo, relapse and complete response samples, relatives of *Bacteroides stercoris* (*Bacteroides sp. DJF_B097*) and *Prevotella copri* (*Prevotella sp. DJF_RP53*) were higher in placebo samples and were associated with complete remission. *B. fragilis* was higher in azithromycin samples and was significantly associated with higher risk of relapse. This higher risk of relapse with *B*. *fragilis* was found with unsupervised multivariate analyses and supervised methods. Longitudinal study of the 3 phylotypes abundances revealed that phylotypes frequencies curves from azithromycin intake overlaid relapse patients curves while placebo overlaid complete remission patients. *B. fragilis* may dampen antitumor responses by promoting regulatory pathways.

Metabolites pathways enriched with phylotypes associated with relapse or remission were (i) phospholipids and lysophospholipids metabolites in patients with lower *Bacteroides sp. DJF_B097,* which may be explained by an impact of azithromycin on these metabolites (Van Bambeke et al. (1996) European Journal of Pharmacology 314, 203-214), (ii) pentose metabolism for *Prevotella sp. DJF_RP53* and (iii) lower BCAA in patients with higher *B. fragilis,* which may be explained by their importance for galactosylceramide biosynthesis (Oh et al. (2021) Nature 600, 302-307).

### SEQUENCES LISTING

denovo9506 of SEQ. ID. N°1 
ACGGGAGGCAGCAGTGAGGAATATTGGTCAATGGGCGCTAGCCTGAACCAGCCAAGTAGCGTGAAGGA
TGAAGGCTCTATGGGTCGTAAACTTCTTTTATATAAGAATAAAGTGCAGTATGTATACTGTTTTGTAT
GTATTATATGAATAAGGATCGGCTAACTCCGTGCCAGCAGCCGCGGTAATACGGAGGATCCGAGCGTT
ATCCGGATTTATTGGGTTTAAAGGGAGCGTAGGTGGACTGGTAAGTCAGTTGTGAAAGTTTGCGGCTC
AACCGTAAAATTGCAGTTGATACTGTCAGTCTTGAGTACAGTAGAGGTGGGCGGAATTCGTGGTGTAG
CGGTGAAATGCTTAGATATCACGAAGAACTCCGATTGCGAAGGCAGCTCACTGGACTGCAACTGACAC
TGATGCTCGAAAGTGTGGGTATCAAACAGGATTAGATACCCTGGTA
denovo3073 of SEQ. ID. N°2 
ACGGGAGGCAGCAGTGAGGAATATTGGTCAATGGACGAGAGTCTGAACCAGCCAAGTAGCGTGAAGGA
TGACTGCCCTATGGGTTGTAAACTTCTTTTATACGGGAATAAAGTTAGCCACGTGTGGCTTTTTGTAT
GTACCGTATGAATAAGGATCGGCTAACTCCGTGCCAGCAGCCGCGGTAATACGGAGGATCCGAGCGTT
ATCCGGATTTATTGGGTTTAAAGGGAGCGTAGGCGGGTTGTTAAGTCAGTTGTGAAAGTTTGCGGCTC
AACCGTAAAATTGCAGTTGATACTGGCGACCTTGAGTGCAACAGAGGTAGGCGGAATTCGTGGTGTAG
CGGTGAAATGCTTAGATATCACGAAGAACTCCGATTGCGAAGGCAGCTTACTGGATTGTAACTGACGC
TGATGCTCGAAAGTGTGGGTATCAAACAGGATTAGATACCCTGGTA
denovo9260 of SEQ. ID. N°3 
ACGGGAGGCAGCAGTGAGGAATATTGGTCAATGGACGAGAGTCTGAACCAGCCAAGTAGCGTGCAGGA
AGACGGCCCTATGGGTTGTAAACTGCTTTTATAAGGGAATAAAGTGAGAGTCGTGACTCTTTTTGCAT
GTACCTTATGAATAAGGACCGGCTAATTCCGTGCCAGCAGCCGCGGTAATACGGAAGGTCCGGGCGTT
ATCCGGATTTATTGGGTTTAAAGGGAGCGTAGGCCGGAGATTAAGCGTGTTGTGAAATGTAGATGCTC
AACATCTGAACTGCAGCGCGAACTGGTTTCCTTGAGTACGCACAAAGTGGGCGGAATTCGTGGTGTAG
 
CGGTGAAATGCTTAGATATCACGAAGAACTCCGATTGCGAAGGCAGCTCACTGGAGCGCAACTGACGC

TGAAGCTCGAAAGTGCGGGTATCGAACAGGATTAGATACCCTGGTA
primers:
   V3fwd-TACGGRAGGCAGCAG wherein R is A or G SEQ ID N°4
   V4rev- TACCAGGGTATCTAAT SEQ ID N°5

## Claims

1. Method for predicting the response of an individual to an allogeneic hematopoietic stem cell transplantation (allo-HSCT) comprising the steps of:
(i) determining gut microbial phylotypes in an intestinal sample of said individual;
(ii) determining an abundance or relative abundance of the phylotypes comprising a nucleotide fragment of sequence having at least 97%, preferably 98%, 99% or 100%, identity with denovo9506 of SEQ. ID. N°1 and optionally determining a relative abundance of at least one of the OTU comprising a nucleotide fragment of sequence having at least 97%, preferably 98%, 99% or 100%, identity with denovo3073 of SEQ. ID. N°2 and/or with denovo9260 of SEQ. ID. N°3;
wherein individual having a gut microbiota enriched in said OTU comprising a nucleotide fragment of sequence having at least 97%, preferably 98%, 99% or 100%, identity with denovo9506 of SEQ. ID. N°1 and optionally depleted in at least one of OTU comprising a nucleotide fragment of sequence having at least 97%, preferably 98%, 99% or 100%, identity with denovo3073 of SEQ. ID. N°2 and/or with denovo9260 of SEQ. ID. N°3 has a higher risk of relapse.

2. Method for predicting the response of an individual to an allogeneic hematopoietic stem cell transplantation (allo-HSCT) according to claim 1, comprising the steps of:
(i) determining gut microbial phylotype in an intestinal sample of said individual;
(ii) determining abundance or relative abundance of the phylotype comprising a nucleotide fragment of sequence having at least 97%, preferably 98%, 99% or 100%, identity with denovo9506 of SEQ. ID. N°1;
wherein individual having a gut microbiota enriched in said OTU has a higher risk of relapse.

3. Method for predicting the response of an individual to an allogeneic hematopoietic stem cell transplantation (allo-HSCT) according to claim 1, comprising the steps of:
(i) determining gut microbial phylotype in an intestinal sample of said individual;
(ii) determining a relative abundance of at least one OTU comprising a nucleotide fragment of sequence having at least 97%, preferably 98%, 99% or 100%, identity with denovo3073 of SEQ. ID. N°2 and/or with denovo9260 of SEQ. ID. N°3;
wherein individual having a gut microbiota enriched in at least one said OTU or in the 2 OTUs is a good responder to the allo-HSCT.

4. Phage targeting directly or indirectly *Bacteroides fragilis* for preventing cancer relapse in a patient having received an allogeneic hematopoietic stem cell transplantation (allo-HSCT).

5. Phage targeting directly or indirectly *Bacteroides fragilis* for use according to claim 4, wherein said phage is selected in the group consisting of lytic phages VA7, MTK and UZ-1, bacteriophage B56-3, bacteriophage B40-8, bacteriophage vB_BfrS_23, *Lactococcus* phage D4410, *Lactococcus* phage D4412, *Lactobacillus* phage Lrm1, *Streptococcus* phage Dp-1, *Rhodococcus* virus Poco6, *Alphapapillomavirus* 10 and *Lactococcus* phage BK5-T.

6. Pharmaceutical composition comprising at least one phage targeting directly or indirectly *Bacteroides fragilis* for preventing cancer relapse in a patient having received an allogeneic hematopoietic stem cell transplantation (allo-HSCT).

7. Pharmaceutical composition comprising at least one phage targeting directly or indirectly *Bacteroides fragilis* for use according to claim 6, wherein said pharmaceutical composition comprises two or more phages targeting directly or indirectly *Bacteroides fragilis.*

8. Pharmaceutical composition comprising at least one phage targeting directly or indirectly *Bacteroides fragilis* for use according to claim 6 or claim 7, wherein said pharmaceutical composition is administered orally or in the form of a suppository.

9. Pharmaceutical composition comprising at least one phage targeting directly or indirectly *Bacteroides fragilis* for use according to claim 8, wherein said pharmaceutical composition is administered orally and further comprises a proton pump inhibitor.

10. Probiotic composition comprising one or more isolated bacterial strains of species selected from the group consisting of *Bacteroides stercoris* and related phylotype *Bacteroides sp. DJF_B097* and/or *Prevotella copri* and related phylotype *Prevotella sp. DJF_RP53* for preventing cancer relapse in a patient having received an allogeneic hematopoietic stem cell transplantation (allo-HSCT).

11. Nutritional composition comprising at least one metabolite selected in the group consisting of group consisting of:
| **metabolite** | **Superpathway** | **Subpathway** |
|---|---|---|
| deoxycholic acid 12-sulfate* | Lipid | Secondary Bile Acid Metabolism |
| deoxycholic acid 3-sulfate | Lipid | Secondary Bile Acid Metabolism |
| 1-methylxanthine | Xenobiotics | Xanthine Metabolism |
| deoxycholate | Lipid | Secondary Bile Acid Metabolism |
| propionylglycine (C3) | Lipid | Fatty Acid Metabolism (also BCAA Metabolism) |
| lithocholate | Lipid | Secondary Bile Acid Metabolism |
| 12-ketolithocholate | Lipid | Secondary Bile Acid Metabolism |
| lithocholic acid sulfate (2) | Lipid | Secondary Bile Acid Metabolism |
| hexadecasphinganine (d16:0)^{∗} | Lipid | Sphingolipid Synthesis |
| ursocholate | Lipid | Secondary Bile Acid Metabolism |
| taurocholenate sulfate^{∗} | Lipid | Secondary Bile Acid Metabolism |
| 3-dehydrodeoxycholate | Lipid | Secondary Bile Acid Metabolism |
| isoursodeoxycholate | Lipid | Secondary Bile Acid Metabolism |
| 1-stearoyl-GPI (18:0) | Lipid | Lysophospholipid |
| butyrylglycine (C4) | Lipid | Fatty Acid Metabolism (also BCAA Metabolism) |
| dehydrolithocholate | Lipid | Secondary Bile Acid Metabolism |
| 1-stearoyl-GPA (18:0) | Lipid | Lysophospholipid |
| glycodeoxycholate 3-sulfate | Lipid | Secondary Bile Acid Metabolism |
| lithocholate sulfate (1) | Lipid | Secondary Bile Acid Metabolism |
| 1-palmitoyl-GPG (16:0)^{∗} | Lipid | Lysophospholipid |
| 1-palmitoyl-GPI^{∗} (16:0) | Lipid | Lysophospholipid |
